# EUROPEAN PATENT APPLICATION

(11) **EP 1 354 551 A1**
(43) Date of publication of application: **22.10.2003**
(21) Application number: 03008735.7
(22) Date of filing: 16.04.2003
(51) Int. Cl.: A61B 3/14

(54) **Ophthalmologic photographing apparatus**

(30) Priority: 19.04.2002 JP 2002118230
(71) Applicant: Kabushiki Kaisha TOPCON, Tokyo 174-8580 (JP)
(72) Inventor: Sugino, Yuichi, Itabashi-ku, Tokyo 174-8580 (JP); Kogawa, Daisaku, Itabashi-ku, Tokyo 174-8580 (JP); Kato, Takeyuki, Itabashi-ku, Tokyo 174-8580 (JP); Abe, Tomoyoshi, Itabashi-ku, Tokyo 174-8580 (JP)
(74) Representative: VOSSIUS & PARTNER

(57) **Abstract**

There is provided an ophthalmologic photographing apparatus in which light from an observation light source or light from a photographing light source is irradiated to an eye to be examined, reflected light from the eye to be examined by the irradiation is guided to a photographing unit having a color area CCD as an image pickup device through an observation optical system in an ophthalmologic photographing apparatus main body, an image of the eye to be examined is imaged onto the color area CCD to photograph the image of the eye to be examined, and the image of the eye to be examined is displayed on an image display device in accordance with an image pickup signal from the image pickup device. The ophthalmologic photographing apparatus has: a light source mode setting switch for setting a combination of the observation light source in observation and the photographing light source in photographing; and a white balance adjusting circuit unit for conducting respective white balance adjustments corresponding to the observation light source and the photographing light source which are set based on setting of the light source mode setting switch.

## Description

The present invention relates to an ophthalmologic photographing apparatus for photographing an eye to be examined by a photographing unit such as a digital still camera and displaying a photographed image of the eye to be examined, thereby observing the eye to be examined to diagnose it.

In general, in an ophthalmologic photographing apparatus for observing the eye to be examined and diagnosing it, an image of the eye to be examined is obtained using an illumination system and an observation system of the ophthalmologic photographing apparatus. The image thus obtained is photographed by a photographing unit such as a digital still camera in which a CCD (charge coupled device) is mounted as an image pickup device, and displayed on an image displaying unit such as a color image monitor. The displayed image is observed by an examiner, and also used for visual identification by a family and the like of a person to be examined.

In such an ophthalmologic photographing apparatus, up to now, in order to obtain a clear image of the eye to be examined with a good color balance in each photographing, a technique of automatic white balance adjustment is generally employed. With the technique, a gain of the image pickup device is automatically controlled every time the image of the eye to be examined is photographed to set an adequate white balance.

Here, the white balance adjustment is a technique of correcting a variation in color tone of an output signal of the CCD according to a color temperature of light from a light source so as not to cause uncomfortable feeling in an eye of an examiner or the like. This becomes an essential function in the case where photographing is conducted by the digital camera using the CCD, or the like.

With respect to an adjusting method for the white balance, a TTL (through the lens) method of computing the amount of correction of color tone using color information of an image taken by the photographing system is mainstream. In addition, a white detection method of extracting a white region from an image and correcting a color difference between the region and another region to "0" is employed in many cases.

Now, in a conventional ophthalmologic photographing apparatus, even when the automatic white balance adjustment is conducted, a high brightness portion and a low brightness portion are mixed in a photographing region of the eye to be examined. Thus, an optimum white balance is not necessarily obtained.

Also, in the ophthalmologic photographing apparatus, switching between an observation light source used for observing the eye to be examined (for example, halogen lamp) and an photographing light source used for photographing the image of the eye to be examined (for example, xenon lamp) is conducted in many cases. Even in such a case, color temperatures of light from both light sources are different from each other. Thus, an optimum white balance is not necessarily obtained only by the conventional white balance technique.

Further, up to now, there is an example in which the white balance is manually adjusted in photographing. However, the fact is that the adjustment of white balance is not conducted in observation.

The present invention has been made in view of the above circumferences. An object of the present invention is to provide an ophthalmologic photographing apparatus capable of obtaining an optimum white balance in respective cases of observation and photographing by automatic operation or simple manual operation, thereby observing and photographing the image of the eye to be examined in a preferable manner.

In order to attain the above-mentioned object, according to a first aspect of the present invention, there is provided an ophthalmologic photographing apparatus in which light from an observation light source or light from a photographing light source is irradiated to an eye to be examined, reflected light from the eye to be examined by the irradiation is guided to a photographing unit having an image pickup device through an observation optical system in an ophthalmologic photographing apparatus main body, an image of the eye to be examined is imaged onto the image pickup device to photograph the image of the eye to be examined, and the image of the eye to be examined is displayed on an image display device in accordance with an image pickup signal from the image pickup device, the ophthalmologic photographing apparatus including: light source mode setting means for setting a combination of the observation light source and the photographing light source in observation and photographing; and white balance adjusting means for conducting respective white balance adjustments corresponding to the observation light source and the photographing light source which are set based on setting of the light source mode setting means.

According to the first aspect of the present invention, the white balance adjustment is automatically conducted corresponding to the observation light source and the photographing light source respectively in accordance with a combination of the observation light source and the photographing light source set by the light source mode setting means. Thus, observation and photographing of the image of the eye to be examined can be conducted under the optimum white balance in a preferable manner.

According to a second aspect of the present invention, there is provided an ophthalmologic photographing apparatus in which light from an observation light source or light from a photographing light source is irradiated to an eye to be examined, reflected light from the eye to be examined by the irradiation is guided to a photographing unit having an image pickup device through an observation optical system in an ophthalmologic photographing apparatus main body, an image of the eye to be examined is imaged onto the image pickup device to photograph the image of the eye to be examined, and the image of the eye to be examined is displayed on an image display device in accordance with an image pickup signal from the image pickup device, the ophthalmologic photographing apparatus including: quantity-of-light setting means for setting the amount of light emission of the photographing light source; means for automatically changing white balance setting in accordance with the amount of light emission of the photographing light source which is set by the quantity-of-light setting means; and white balance adjusting means for conducting white balance adjustment corresponding to the changed white balance setting.

According to the second aspect of the present invention, the white balance setting is automatically switched in accordance with the set amount of light of the photographing light source, and the white balance adjustment corresponding to the switched setting is conducted. Thus, for example, even when the amount of light is changed to conduct photographing or even when non-flash photographing is conducted, observation and photographing of the image of the eye to be examined can be conducted under the optimum white balance in a preferable manner.

According to a third aspect of the present invention, there is provided an ophthalmologic photographing apparatus including: an ophthalmologic photographing apparatus main body having an observation light source for irradiating light from the observation light source to an eye to be examined and observing reflected light from the eye to be examined by the irradiation through an observation optical system; a photographing unit which has an image pickup device and a photographing light source and is integrated with the ophthalmologic photographing apparatus main body; an image display device for displaying an observation image in the observation optical system; means for automatically changing white balance setting for each light source in observation and photographing in accordance with a type of the observation light source of the ophthalmologic photographing apparatus main body and a type of the photographing light source of the photographing device; and white balance adjusting means for conducting white balance adjustment in observation and photographing corresponding to the changed white balance setting.

According to the third aspect of the present invention, even in the case of an ophthalmologic photographing apparatus to which an external photographing device is attached, observation and photographing of the image of the eye to be examined can be conducted under the optimum white balance in a preferable manner.

In the accompanying drawings:
Fig. 1 is an optical system diagram showing a structure of an eye fundus camera according to Embodiment 1 of the present invention;
Fig. 2 is a block diagram showing a structure of a control system in the eye fundus camera according to Embodiment 1;
Fig. 3 is a schematic side view of the eye fundus camera according to Embodiment 1;
Fig. 4 is a table showing combination examples of types of light sources in the eye fundus camera according to Embodiment 1;
Fig. 5 is a partial block diagram showing a white balance adjusting unit according to Embodiment 2 of the present invention;
Fig. 6 is a partial block diagram showing a white balance adjusting unit according to Embodiment 3 of the present invention; and
Fig. 7 shows a light source mode setting screen by software processing when combination setting operation of an observation light source and a photographing light source, one-push operation, and color temperature setting operation are conducted according to Embodiments 1 to 3 of the present invention.

Hereinafter, embodiments of the present invention will be described in detail.

### (Embodiment 1)

### [Schematic Entire Structure]

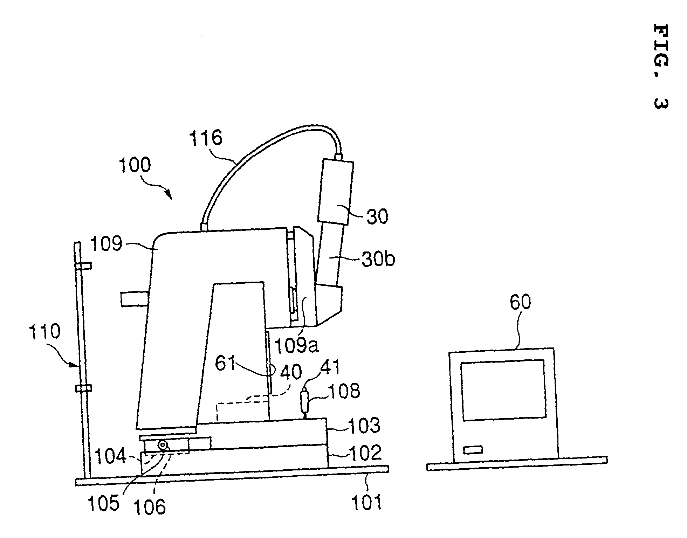

In Fig. 3, reference numeral 100 denotes an eye fundus camera as an ophthalmologic photographing apparatus of Embodiment 1. The eye fundus camera 100 includes a base 101 (pedestal), a fixed table 102 fixed on the base 101, and a movable table 103 placed on the fixed table 102.

As shown in Fig. 3, a pair of guide rails 104, 104 laterally spaced apart from each other are provided on the fixed table 102. In addition, a rotation shaft 105 whose both ends each are laterally extended to the guide rails 104, 104 and provided so as to be rotatable about a shaft line, and wheels 106, 106 which are fixed to both ends of the rotation shaft 105 and guided by the guide rails 104, 104 are provided to the movable table 103. The movable table 103 is held to the rotation shaft 105 so as to be movable in the lateral direction. A rotary encoder 107 for detecting the amount of longitudinal movement in the direction (longitudinal position detecting unit) is interposed between the movable table 103 and the rotation shaft 105.

The eye fundus camera 100 includes a joystick lever 108 which is attached to the movable table 103 so as to be moveable in a tilt state in an arbitrary direction and used for conducting movement operation for the movable table 103 in a tilt movement direction at tilt movement operation, and a photographing switch 41 attached to the joystick lever 108.

Further, the eye fundus camera 100 includes a camera main body 109 (ophthalmologic photographing apparatus main body) fixed on the moveable table 103 and a digital camera 30 as a color photographing unit which is attached to a camera mount 109a through an attachment 30b. The camera mount 109a is detachably attached to the camera main body 109. In Fig. 3, reference numeral 116 denotes a code connecting the digital camera 30 with a control circuit 40 of the camera main body 109. In addition, reference numeral 110 denotes a chin rest stand for holding the chin of a person to be examined.

### [Optical System in Camera Main Body 109]

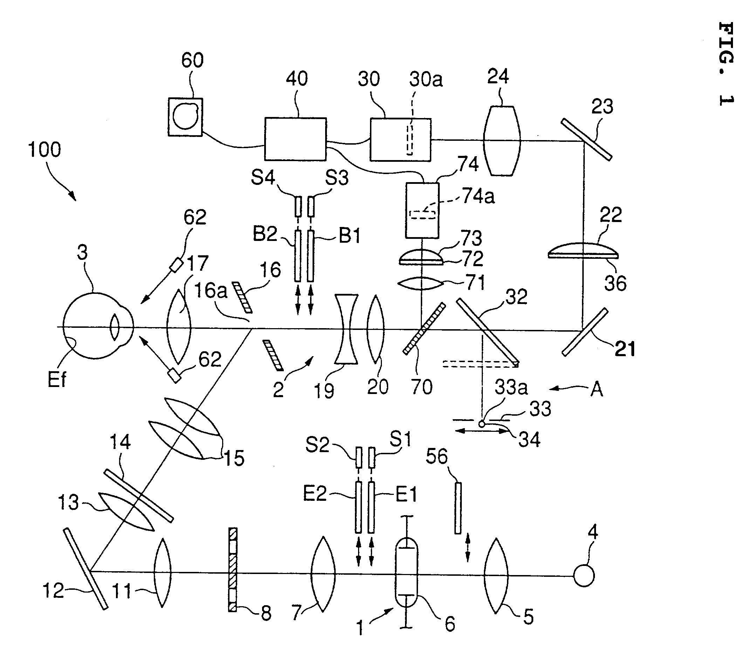

In Fig. 1, reference numeral 1 denotes an illumination optical system of the camera main body 109 in the eye fundus camera 100 of Embodiment 1, 2 denotes an observation and photographing optical system provided in the camera main body 109, and 3 denotes an eye to be examined.

The illumination optical system 1 includes an observation illumination optical system and a photographing illumination optical system. In other words, the illumination optical system 1 includes optical parts such as a ring-shaped diaphragm 8, a relay lens 11, a reflection mirror 12, a relay lens 13, a black spot plate 14, relay lenses 15, a holed mirror 16, and an objective lens 17, which are disposed in this order. Photographing illumination light from a xenon lamp 6 as a photographing light source is irradiated to an eye fundus Ef of the eye to be examined 3 through an optical path of from a condenser lens 7 to the objective lens 17.

A visible fluorescent exciter filter E1 and an infrared fluorescent exciter filter E2 are disposed between the xenon lamp 6 and the condenser lens 7 so as to be removably inserted in the optical path by solenoids S1 and S2 serving as first driver units.

The observation illumination optical system of the illumination optical system 1 is composed of optical parts such as a halogen lamp 4 as an observation light source, a condenser lens 5, and the condenser lens 7 to the objective lens 17. Observation illumination light from the halogen lamp 4 is irradiated to the eye fundus Ef through the optical parts of from the condenser lens 7 to the objective lens 17. Further, in the illumination optical system 1, LEDs 62 for irradiating to the eye 3 to be examined light for background illumination of an observation region are arranged in the vicinity of the objective lens 17. In Fig. 1, reference numeral 56 denotes a shutter member.

### [Observation and Photographing Optical System]

The observation and photographing optical system 2 has a color photographing optical system and an infrared photographing optical system. The color photographing optical system has an internal optical system provided in the camera main body 109 and an external optical system provided in the digital camera 30 side connected with the camera main body 109.

The internal optical system includes the objective lens 17 facing the eye to be examined, a focusing lens 19, and an imaging lens 20. The external optical system includes a mirror 21, a mask 36, a field lens 22 conjugate with the eye fundus Ef, a reflection mirror 23, a relay lens 24, and the digital camera 30. In Fig. 1, 30a denotes a color area CCD as a color image pickup device which is mounted in the digital camera 30.

The infrared photographing optical system includes the objective lens 17, the focusing lens 19, the imaging lens 20, a dichroic mirror 70 disposed between the imaging mirror 20 and the mirror 21, a lens 71, a mask 72, a field lens 73, and an infrared photographing camera 74 sensitive to the infrared light. The dichroic mirror 70 is constructed so as to reflect light having an infrared region wavelength and transmit light having visible light region wavelength. In Fig. 1, 74a denotes an area CCD of the infrared photographing camera 74 which is sensitive to the infrared light.

A visible fluorescent barrier filter B1 and an infrared fluorescent barrier filter B2 are removably disposed between the holed mirror 16 and the focusing lens 19 so as to be inserted in the optical path by solenoids S3 and S4.

According the above-mentioned structure, a color eye fundus image resulting from visible reflection light from the eye fundus Ef of the eye 3 to be examined in the case of using a mydriatic agent is imaged onto the area CCD 30a of the digital camera 30 through the optical parts of from the objective lens 17 to the imaging lens 20, the mirror 21, the field lens 22 conjugate with the eye fundus Ef, the reflection mirror 23, the relay lens 24, and the mask 36.

On the other hand, an infrared eye fundus image resulting from infrared reflection light from the eye fundus Ef of the eye 3 to be examined in the case of not using a mydriatic agent is imaged onto the area CCD 74a of the infrared photographing camera 74 through the optical parts of from the objective lens 17 to the imaging lens 20, the dichroic mirror 70, the lens 71, the mask 72 and the field lens 73.

### [Fixed Target Optical System]

A fixed target optical system "A" has a half mirror 32 pivotably disposed so as to be inserted in the optical path between the imaging lens 20 and the mirror 21, a fixed target mask 33 which has a small hole 33a as a fixed target and is disposed conjugate with the eye fundus E, and a fixed target light source 34 such as a light emitting diode which is disposed to face the small hole 33a.

Note that the mask 33 and the fixed target light source 34 are installed to a fixed target driver device (not shown) such as an X-Y table which is electrically controlled by a pulse motor 108 and the like.

### [Control System]

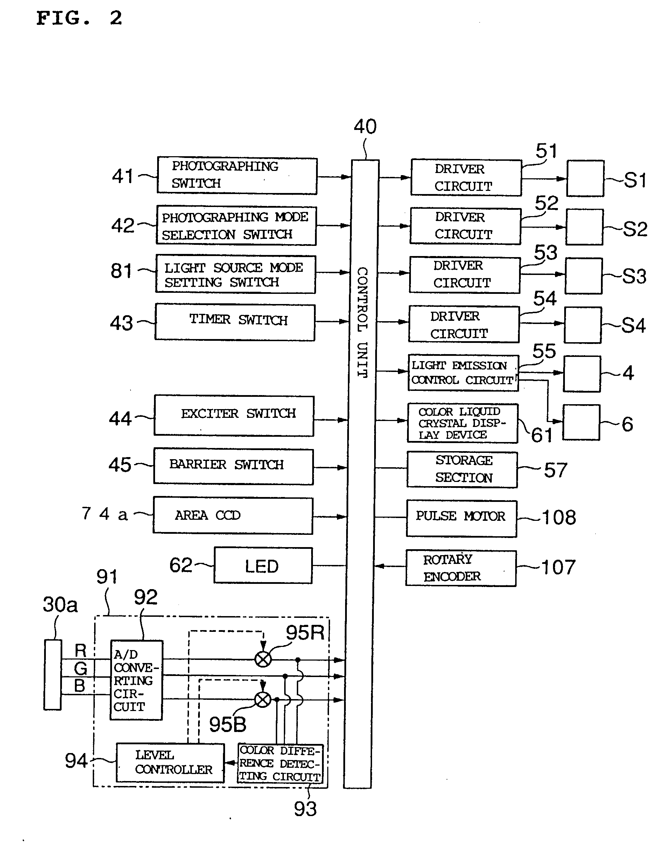

Further, the eye fundus camera 100 includes a control system as shown in Fig. 2.

The control system has the control circuit 40 for controlling the entire units and is constructed such that signals from the photographing switch 41, a photographing mode selection switch 42, a light source mode setting switch 81, a timer switch 43, an exciter switch 44, a barrier switch 45, the rotary encoder 107, and the like are inputted to the control circuit 40.

The control circuit 40 is constructed so as to drive and control the solenoids S1, S2, S3 and S4 through driver circuits 51, 52, 53 and 54 and conduct light emission controls to the xenon lamp 6 and the halogen lamp 4 through a light emission control circuit 55.

Further, the control circuit 40 conducts display control of a color liquid crystal display device 61 included in the camera main body 109. In addition, the control circuit 40 is connected with a color monitor 60 which is placed separate from the camera main body 109 and conducts color image display.

Furthermore, the control circuit 40 is connected with the area CCD 74a of the infrared photographing camera 74 and connected with the area CCD 30a of the digital camera 30 through a white balance adjusting circuit 91.

The white balance adjusting circuit 91 has an A/D converting circuit 92 for conducting A/D conversion to respective output signals of R, G and B of the area CCD 30a, a color difference detecting circuit 93 for detecting color differences (R-G and B-G) based on the A/D converted respective output signals of R, G and B using a green (G) signal as a reference, and a level controller 94 for controlling a level regulator 95R for a red (R) signal line and a level regulator 95B for a blue (B) signal line, except for a green (G) signal line, based on a detection result of the color difference detecting circuit 93, to control the respective signal levels, thereby optimizing a white balance.

The photographing mode selection switch 42 is used for selection among a visible color electronic photographing mode, a visible fluorescent electronic photographing mode, an infrared electronic photographing mode, an infrared fluorescent electronic photographing mode and the like.

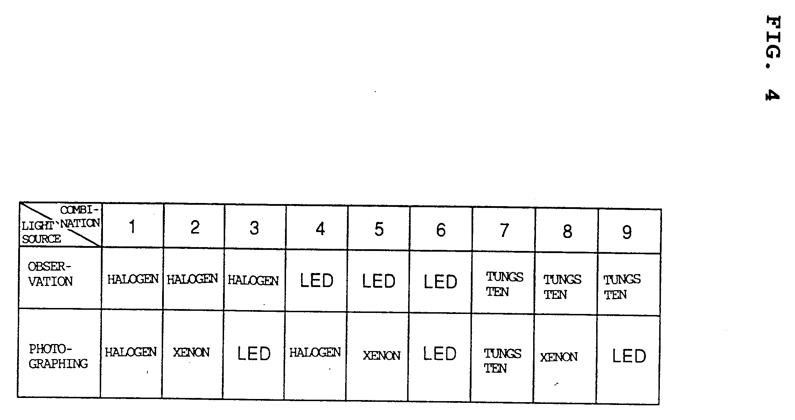

The light source mode setting switch 81 is used for setting a combination of any one of the observation light sources and any one of the photographing light sources in observing and photographing the eye 3 to be examined by, for example, a plurality of button operations. As shown in Fig. 4, three types: a halogen lamp, an LED and a tungsten lamp are theoretically assumed as the observation light sources, and four types: the halogen lamp, a xenon lamp, the LED and the tungsten lamp are theoretically assumed as the photographing light sources.

In Embodiment 1, an examiner sets, for example, a combination of the halogen lamp 4 as the observation light source and the halogen lamp 4 as the photographing light source (combination 1) or a combination of the halogen lamp 4 as the observation light source and the xenon lamp 6 as the photographing light source (combination 2) by the light source mode setting switch 81.

The combination of the observation light source and the photographing light source is changed according to respective ophthalmologic photographing apparatuses. An apparatus in which any one of the halogen lamp, the LED, and the tungsten lamp is mounted as the observation light source and any one of the halogen lamp, the LED, and the tungsten lamp is mounted as the photographing light source is assumed. In this case, the combinations of the observation light source and the photographing light source correspond to combinations 3 to 9 shown in Fig. 4.

The combination of the observation light source and the photographing light source which is set by the light source mode setting switch 81 is stored in a storage section 57.

Next, the operation of the eye fundus camera 100 of Embodiment 1 will be described by referring to mainly white balance adjusting operation according to a type of the observation light source and that of the photographing light source.

### (Automatic White Balance Adjustment)

When a power source switch not shown is turned on, the control circuit 40 operates to conduct initialization operation. Then, the halogen lamp 4 of the illumination optical system 1 is turned on to illuminate the eye 3 to be examined. Next, focusing operation to the eye fundus Ef is conducted by the observation and photographing optical system.

After the completion of the focusing operation, when the photographing switch 41 is turned on by an examiner, the control circuit 40 controls the light emission control circuit 55 so that the halogen lamp 4 is turned off or blinked and the xenon lamp 6 emits light. Thus, the photographing illumination light from the xenon lamp 6 is irradiated to the eye fundus Ef of the eye 3 to be examined through the illumination optical system 1 to illuminate the eye fundus Ef.

The visible reflection light from the eye fundus Ef is incident into the area CCD 30a of the digital camera 30 through the optical parts of from the objective lens 17 to the relay lens 24 to image an eye fundus image for photographing. In addition, the eye fundus image is displayed on the color monitor 60 and used for observation.

In the case of conducting such observation and photographing of the eye fundus image, when the examiner sets the combination of the observation light source and the photographing light source by the light source mode setting switch 81, information related to the combination is stored in the storage section 57. For example, when the examiner sets the combination of the observation light source and the photographing light source to the combination 2 by the light source mode setting switch 81, the observation light source is set to the halogen lamp 4 and the photographing light source is set to the xenon lamp 6. Then, information related to the combination is stored in the storage section 57. In addition, the observation of the eye fundus image using light from the halogen lamp 4 and the photographing of the eye fundus image using light from the xenon lamp 6 are conducted.

In this case, when the eye fundus image is observed, the level controller 94 of the white balance adjusting circuit 91 refers the detection result of the color difference detecting circuit 93 under the control of the control circuit 40 and controls the level regulator 95R for the red (R) signal line and the level regulator 95B for the blue (B) signal line to adjust the respective signal levels so as to correspond to the halogen lamp 4, thereby optimizing a white balance of the eye fundus image in observation.

When the photographing switch 41 is turned on by the examiner, the level controller 94 of the white balance adjusting circuit 91 refers the detection result of the color difference detecting circuit 93 under the control of the control circuit 40 and controls the level regulator 95R for the red (R) signal line and the level regulator 95B for the blue (B) signal line to adjust the respective signal levels so as to correspond to the xenon lamp 6, thereby optimizing a white balance of the eye fundus image in photographing.

For example, when the examiner sets the combination of the observation light source and the photographing light source to the combination 1 by the light source mode setting switch 81, the observation light source is set to the halogen lamp 4 and the photographing light source is also set to the halogen lamp 4. Then, information related to the combination is stored in the storage section 57. In addition, the observation of the eye fundus image using light from the halogen lamp 4 and the photographing of the eye fundus image also using light from the halogen lamp 4 are conducted (non-flash photographing).

In this case, when the eye fundus image is observed and photographed, the level controller 94 of the white balance adjusting circuit 91 refers the detection result of the color difference detecting circuit 93 under the control of the control circuit 40 and controls the level regulator 95R for the red (R) signal line and the level regulator 95B for the blue (B) signal line to adjust the respective signal levels so as to correspond to the halogen lamp 4.

As a result, observation and photographing of the eye fundus image under the optimum white balance can be conducted in a preferable manner. In particular, it is suitable for the case where the examiner, a family of the person to be examined, or the like views the color monitor 60 and observes the eye fundus image.

Even in the case of an ophthalmologic photographing apparatus in which light sources corresponding to the respective combinations 3 to 9 shown in Fig. 4 are mounted, white balances are adjusted for each combination as described above, thereby optimizing the white balances in observing and photographing the eye fundus image according to the types of the respective light sources.

### (Embodiment 2)

### [One-Push White Balance Adjustment]

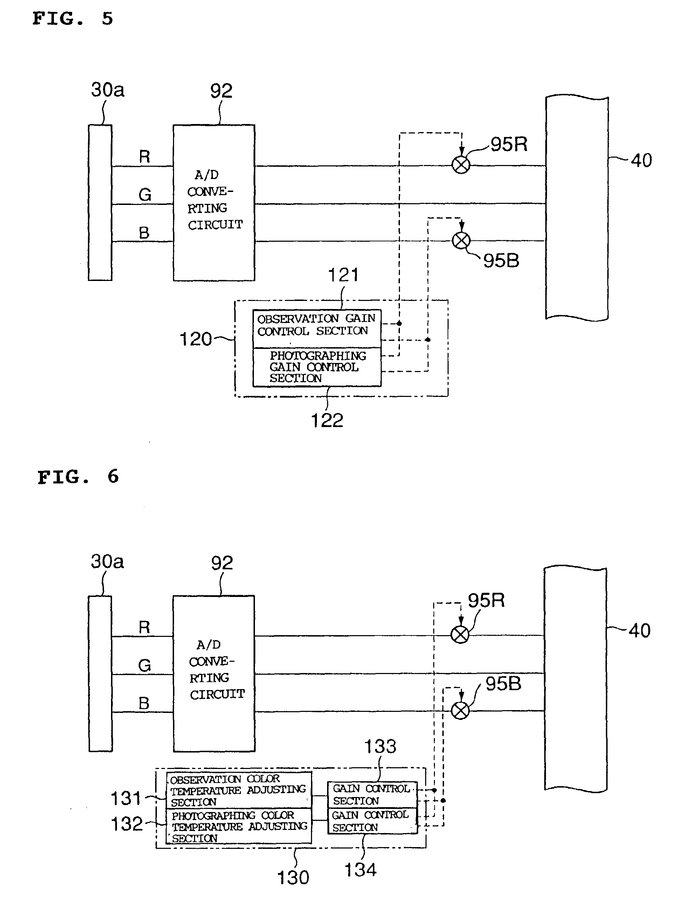

Next, the case where one-push white balance adjustment is conducted according to Embodiment 2 will be described with reference to Fig. 5. Here, one-push means that a white paper is photographed by an observation light source and a photographing light source which are used in advance, thereby obtaining an image.

In this case, the examiner uses a halogen lamp 4 and photographs an image of a white paper with a state in which the white paper is set in a position corresponding to an eye 3 to be examined. Then, while the image of the white paper is referred to, the level regulator 95R for the red (R) signal line and the level regulator 95B for the blue (B) signal line are controlled by an observation gain control section 121 of a white balance manual adjusting unit 120 to optimize a white balance of an eye fundus image in observation. Similarly, the examiner uses a xenon lamp 6 and photographs the image of the white paper with the state in which the white paper is set in the position corresponding to the eye 3 to be examined. Then, while the image of the white paper is referred to, the level regulator 95R for the red (R) signal line and the level regulator 95B for the blue (B) signal line are controlled by a photographing gain control section 122 of the white balance manual adjusting unit 120 to optimize a white balance of an eye fundus image in photographing.

Even in such one-push white balance adjustment, respective white balances of the eye fundus image in observation and photographing can be optimized.

### (Embodiment 3)

### [Color Temperature Adjustment for Light Source]

White balance adjustment by color temperature adjustment for a light source according to embodiment 3 will now be described with reference to Fig. 6.

In this case, as shown in Fig. 6, a color temperature adjusting unit 130 is composed of an observation color temperature adjusting section 131 and a photographing color temperature adjusting section 132, which are used for setting a color temperature value according to a plurality of button operations or the like by an examiner, and gain control sections 133 and 134 for changing a red gain and a blue gain of an image pickup device according to the respective set color temperature values. The level regulator 95R for the red (R) signal line and the level regulator 95B for the blue (B) signal line are controlled according to color temperature values adjusted by the gain control sections 133 and 134 to optimize white balances of an eye fundus image in observation and photographing.

An adjustment range of the color temperature value is, for example, 3000K (Kelvin) to 5500K. When a halogen lamp 4 is used, the value is set to about 3000K. When a xenon lamp 6 is used, the value is set to about 5000K. When LEDs 62 are used, the value is set to about 5500K.

Even in such white balance adjustment by color temperature adjustment according to the types of light sources in Embodiment 3, respective white balances of the eye fundus image in observation and photographing can be optimized.

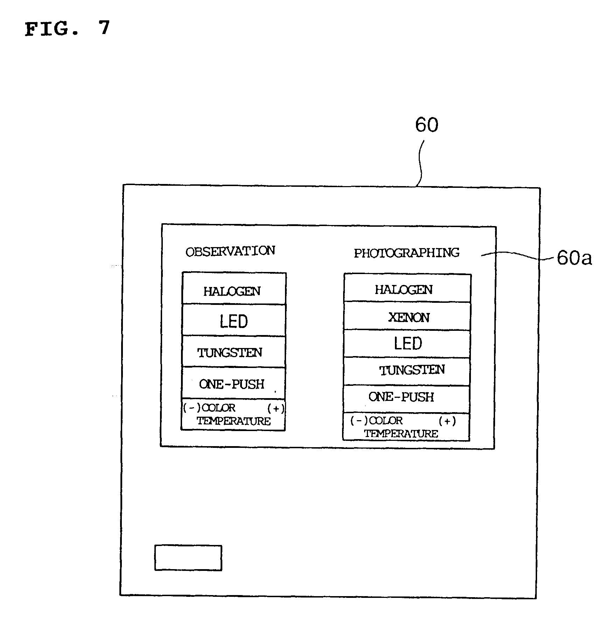

Fig. 7 shows a light source mode setting screen 60a by software processing when the combination setting operation of the observation light source and the photographing light source as shown in Fig. 4, one-push operation, and color temperature setting operation are conducted. Names of plural types of observation light sources and photographing light sources in observation and photographing, one-push operation, respective names of color temperatures (plus (+) and minus (-)) are displayed on the screen in a list form by software processing. Thus, combination setting operation of light sources in observing and photographing the eye fundus image, one-push operation, and color temperature setting operation (plus or minus) can be conducted according to mouse click operation by the examiner.

In Embodiments 1 to 3 as described above, the eye fundus 100 is described as an example. The present invention can be applied to an ophthalmologic photographing apparatus using a slit lamp microscope (slit lamp).

With respect to a structure of the ophthalmologic photographing apparatus in this case, a digital camera as a photographing unit is attached to the slit lamp microscope, and a color monitor is further provided thereto. As in Embodiment 1, the combination of the observation light source and the photographing light source is set according to the types of light sources mounted in the slit lamp microscope. Thus, observation and photographing of a slit image of the cornea of the eye to be examined can be conducted under the respective optimum white balance settings.

In particular, it is suitable for the case where the examiner, a family of the person to be examined, or the like views the color monitor and observes the slit image in real time.

According to the embodiments as described above, the eye fundus 100 is described in which the observation light source and the photographing light source are mounted. In addition to this, for example, when the amount of light of the xenon lamp for photographing can be adjusted from 0 to a maximum level by a changing switch or the like, and except for the case of setting of 0 level in which light is not emitted from the xenon lamp (light emission from xenon lamp), white balance setting for xenon lamp in photographing can automatically be conducted using a setting signal from the changing switch. In addition, when a light emission level of the xenon lamp is set to 0 level by the examiner (non-flash), it can automatically be changed to white balance setting for halogen lamp which is used for observation and photographing using the setting signal. After changing, the same white balance adjustment as in the above-mentioned case is conducted.

Also, white balance setting for xenon lamp in each photographing can automatically be conducted according to the different amounts of light emission (for example 100 W, 300 W, and the like) using the setting signal from the changing switch. After changing, the same white balance adjustment as in the above-mentioned case is conducted.

Further, as another application example, there is a case where an ophthalmologic photographing apparatus is constructed by attaching a digital camera as a photographing device to an ophthalmologic apparatus such as a slit lamp.

In this case, an image of the eye to be examined is observed and photographed by using a combination of an observation light source in the ophthalmologic apparatus side and a photographing light source in the digital camera side. However, the type of the photographing light source in the digital camera side is sent to an ophthalmologic apparatus main body by transmission of a signal indicating the light source type through terminal connection for signal transmission between the digital camera and the ophthalmologic apparatus. Thus, white balance setting for each light source in observation and photographing can automatically be changed in the ophthalmologic apparatus main body side. After changing, the same white balance adjustment as in the above-mentioned case is conducted.

According to the first aspect of the present invention, optimal white balance is automatically set according to setting of light source combination by the light source mode setting unit. Thus, the ophthalmologic photographing apparatus capable of observing and photographing the image of the eye to be examined in a preferable manner can be provided.

According to the second aspect of the present invention, even in the case of non-flash photographing, the ophthalmologic photographing apparatus capable of observing and photographing the image of the eye to be examined under optimum white balance in a preferable manner can be provided.

According to the third aspect of the present invention, even in the case of an ophthalmologic photographing apparatus to which an external photographing device is attached, the ophthalmologic photographing apparatus capable of observing and photographing the image of the eye to be examined under optimum white balance in a preferable manner can be provided.

## Claims

1. An ophthalmologic photographing apparatus in which light from an observation light source or light from a photographing light source is irradiated to an eye to be examined, reflected light from the eye to be examined by the irradiation is guided to a photographing unit having an image pickup device through an observation optical system in an ophthalmologic photographing apparatus main body, an image of the eye to be examined is imaged onto the image pickup device to photograph the image of the eye to be examined, and the image of the eye to be examined is displayed on an image display device in accordance with an image pickup signal from the image pickup device, the ophthalmologic photographing apparatus comprising:
light source mode setting means for setting a combination of the observation light source and the photographing light source in observation and photographing; and
white balance adjusting means for conducting respective white balance adjustments corresponding to the observation light source and the photographing light source which are set based on setting of the light source mode setting means.

2. An ophthalmologic photographing apparatus in which light from an observation light source or light from a photographing light source is irradiated to an eye to be examined, reflected light from the eye to be examined by the irradiation is guided to a photographing unit having an image pickup device through an observation optical system in an ophthalmologic photographing apparatus main body, an image of the eye to be examined is imaged onto the image pickup device to photograph the image of the eye to be examined, and the image of the eye to be examined is displayed on an image display device in accordance with an image pickup signal from the image pickup device, the ophthalmologic photographing apparatus comprising:
quantity-of-light setting means for setting the amount of light emission of the photographing light source;
means for automatically changing white balance setting in accordance with the amount of light emission of the photographing light source which is set by the quantity-of-light setting means; and
white balance adjusting means for conducting white balance adjustment corresponding to the changed white balance setting.

3. An ophthalmologic photographing apparatus comprising:
an ophthalmologic photographing apparatus main body having an observation light source for irradiating light from the observation light source to an eye to be examined and observing reflected light from the eye to be examined by the irradiation through an observation optical system;
a photographing unit which has an image pickup device and a photographing light source and is integrated with the ophthalmologic photographing apparatus main body;
an image display device for displaying an observation image in the observation optical system;
means for automatically changing white balance setting for each light source in observation and photographing in accordance with a type of the observation light source of the ophthalmologic photographing apparatus main body and a type of the photographing light source of the photographing device; and
white balance adjusting means for conducting white balance adjustment in observation and photographing corresponding to the changed white balance setting.
